# EUROPEAN PATENT APPLICATION

(11) **EP 0 956 850 A1**
(43) Date of publication of application: **17.11.1999**
(21) Application number: 98201275.9
(22) Date of filing: 21.04.1998
(51) Int. Cl.: A61K 7/00, A61K 7/06, A61K 7/50

(54) **Hair care products containing an ehec ether**

(71) Applicant: Akzo Nobel N.V., 6824 BM Arnhem (NL)
(72) Inventor: Krüger, Götz, 52074 Aachen (DE); Plate, Holger, 52379 Langerwehe (DE); Tang, Yonghua, Albertson, NY 11507 (US)
(74) Representative: Schalkwijk, Pieter Cornelis, c.s.

(57) **Abstract**

The present invention relates to a hair care formulation, such as a hair conditioner, comprising a quaternary ammonium compound and an ethyl hydroxyethyl cellulose ether. The quaternary ammonium compound preferably is a monoalkylquat, a dialkylquat or a diesterquat. When applied on hair, the formulations according to the present invention show improved consistency and offer improved wet- and dry-combing and feel.

## Description

The present invention relates to a hair care formulation.

Hair care formulations, e.g. hair conditioners, containing a quaternary ammonium compound (quat) and a cellulose ether are commercially available, e.g. Pantene Pro V® from Procter & Gamble, Nivea Haarpflege® from Beiersdorf AG, and Guhl® from Guhl Ikebana Kosmetik. In all of these prior art formulations hydroxyethyl cellulose is used, which functions mainly as a thickening agent enhancing the viscosity of the formulation. In order to further improve the conditioning properties of such formulations surface-active cellulose ethers have been incorporated, e.g. in Poly kur® from Henkel Cosmetic, which contains hydroxypropyl methyl cellulose (HPMC). Due to its surface activity HPMC also acts as a stabilizer of the foam that is created by using a shampoo containing HPMC. The conditioning properties and compatability with surfactant systems of this surface-active cellulose ether, however, are not yet optimal. Hence, in the field of hair care products there still is a need for new cellulose ether products having good overall conditioning properties and with good surfactant compatability, which allow the preparation of economical formulations.

The hair care formulation according to the present invention comprising a quaternary ammonium compound and a cellulose ether is characterized in that the cellulose ether is an ethyl hydroxyethyl cellulose ether.

Preferably, the ethyl hydroxyethyl cellulose ether comprised in the hair care formulation of the present invention is ethyl hydroxyethyl cellulose (EHEC). A wide range of EHEC ethers are commercially available in a variety of molecular weights, providing viscosity enhancements from approximately 1,000 mPa.s to in excess of 50,000 mPa.s, for a 1 weight percent aqueous solution. A very suitable EHEC ether is Elfacos® CD 481 (ex. Akzo Nobel). Hydrophobically modified EHEC ethers may also be favourably employed in the hair care formulations of the present invention.

The ethyl hydroxyethyl cellulose ether that is used in the hair care formulation of the invention typically has an ethylene oxide degree of molar substitution (MS_{EO}) of 1.5 to 3.5, an ethyl degree of substitution (DS_{Et}) of 0.5 to 1.5, and an average degree of polymerization (DP) of 500 to 2,500. Preferably, it has a MS_{EO} of 2.0-3.0, a DS_{Et} of 0.7-1.2, and a DP of 1,000-2,000, more preferably a MS_{EO} of 2.2-2.9, a DS_{Et} of 0.8-1.0, and a DP of 1,500-2,500.
The ethyl hydroxyethyl cellulose ethers that are used in the hair care formulations of the invention typically lower the surface tension of aqueous solutions to levels below 70, preferably below 60, more preferably below 55 mN/m at 25°C at a concentration of typically about 0.2 weight percent.
Also, the interfacial tension of aqueous solutions versus oils is lowered.
They further typically show a cloud point (i.e. phase transition temperature) in aqueous solutions of 50-95°C, preferably of 55-75°C, e.g. Elfacos® CD 481 shows a cloud point of approximately 63°C. Surface tension, interfacial tension, and cloud point can be determined by any method known to the person skilled in the art. For example, the cloud point can be determined by turbidity measurement.
Typically, an amount of 0.1 to 3.0 weight percent of the ethyl hydroxyethyl cellulose ether, based on the total weight of the formulation, is used in the hair care formulation of the invention. Preferably, an amount of 0.5 to 1.5 weight percent is employed. Most preferably, an amount of 0.5 to 1.0 weight percent is incorporated into the formulation according to the present invention.

The quaternary ammonium compounds (quats) that can be used in the hair care formulations according to the present invention are known to the person skilled in this art. Typical examples of suitable quats include:
monoalkylquats of the formula RN(Me)₃⁺;
dialkylquats of the formula R₂N(Me)₂⁺;
monoesterquats of the formula RC(O)OCH₂CH₂N(Me)₂(CH₂CH₂OH)⁺;
monoesterquats of the formula RC(O)OCH₂CH₂N(Me)(CH₂CH₂OH)₂⁺;
monoesterquats of the formula RC(O)OCH₂CH₂N(Me)₃⁺;
diesterquats of the formula (RC(O)OCH₂CH₂)₂N(Me)₂⁺;
diesterquats of the formula (RC(O)OCH₂CH₂)₂N(Me)(CH₂CH₂OH)⁺;
   wherein R is a C₁₂-C₂₂ alkyl or a C₁₂-C₂₂ alkenyl group.
Preferably, R is a C₁₆-C₂₂ alkyl or a C₁₆-C₂₂ alkenyl group, most preferably a C₁₆ alkyl or a C₁₆ alkenyl group.

Either a single quat or a mixture of the aforementioned quats can be used.
A typical example of a suitable alkylquat is cetyl trimethylammonium chloride or cetrimonium chloride (i.e. Arquad® 16-25, ex. Akzo Nobel) and of a diesterquat is dipalmitoylethyldimonium chloride (i.e. Armocare® VGH-70, ex. Akzo Nobel).
Typically, an amount of 0.15 to 5 weight percent of the quaternary ammonium compound (i.e. active ingredient), based on the total weight of the formulation, is used in the hair care formulation of the invention. It is preferred to employ an amount of 0.2 to 4, more preferably 0.5 to 3, weight percent of the quat.
As will be appreciated by the skilled person, besides the quaternary ammonium compound and the surface-active cellulose ether, the hair care formulation according to the present invention may contain one or more ingredients selected from fatty alcohols, e.g. cetyl alcohol and cetearyl alcohol, emulsifiers, oil components, organic acids to control the pH, e.g. citric acid, humectants, like glycerin or other polyols such as glycols, anti-dandruffs, UV absorbers, vitamins, dyes, and/or fragrances.

The hair care formulations according to the present invention can be prepared by methods which are known per se. They can, for example, be prepared by mixing all ingredients or by mixing separate premixes. These premixes may have been prepared at different temperatures in order to achieve dissolution and/or prevent degradation of the ingredients.
For example, monoalkylquats and monoesterquats are liquids and can be incorporated at room temperature. The diesterquat Armocare® VGH-70 can also be incorporated at room temperature, while dialkylquats can be conveniently dissolved at approximately 70°C. In general, esterquats are sensitive to hydrolysis and are therefore best incorporated at low temperature and low pH. If fatty alcohols are used, they are suitably dissolved at temperatures of approximately 60-70°C.

For further details on quats and optional ingredients that can be used, as well as on suitable preparative methods, the reader is referred to the following documents: "The influence of chemical structure on performance in hair care preparations" by E. Spiess in Parfümerie und Kosmetik, 72, 1991, pages 370-376; "Quaternary ammonium salt" by M.F. Jurczyk, D.R. Berger, and G.R. Damaso in Cosmetics & Toiletries, 106, 1991, pages 63-68; "Eigenschaften und Anwendung kationischer Haarpflegeadditive" by J. Koester in Parfümerie und Kosmetik, 72, 1991, pages 218-225; Handbook of Cosmetic Science and Technology by J. Knowlton and S. Pearce, 1st edition, 1993, Elsevier advanced technology, Oxford, pages 15-17 and 226; Harry's Cosmeticology, Edited by J.B. Wilkinson and R.J. Moore, seventh edition, 1982, Longman Scientific & Technical, Essex, pages 513-518; Cosmetic and Toiletry Formulations by E. W. Flick, second edition, Volume 3, 1995, Noves Publications, New Jersey, U.S.A.; Kosmetik by W. Umbach, 1988, Georg Thieme Verlag Stuttgart, New York; Grundlagen und Rezepturen der Kosmetika by K. Schrader,1989, Hüthig Buch Verlag Heidelberg, pages 722-737; Product sheet Armocare® VGH-70, Akzo Nobel Surface Chemistry, 1995; "Water-soluble cellulose ethers for hair care products" by M.T. Clarke in Seifen - Öle - Fette - Wachse, 116, 1990, pages 684-687; and "Hydrophobically modified cellulose ether for hair care" by J.J. de Bruin in SÖFW-Journal, 120, 1994, pages 944-948.

The hair care formulations of the present invention typically are aqueous liquids which can be applied to the hair as clear rinses, cream rinses (i.e. lotions, emulsions) or mousses.

The present invention is illustrated by the following Examples.

Ethyl hydroxyethyl cellulose, Elfacos® CD 481, ex. Akzo Nobel:

| | |
|---|---|
| Appearance | off-white powder |
| Particle size | 98% <425 µm |
| Sodium chloride | ≤ 4% |
| Water | ≤ 4% |
| pH (1% in water) | 6.0-7.0 |
| Viscosity (1% in water) | 4000-6000 mPa.s (Brookfield LV, spindle no. 3, 12 rpm) |

A typical hair care formulation, i.e. hair conditioner, contains the following ingredients (in weight percent):

| | |
|---|---|
| Elfacos® CD 481 | 0.7 |
| Cetrimonium chloride (Arquad® 16-25) | 3.0 |
| Cetearyl alcohol (Laureth-10) | 0.5 |
| Isopropyl stearate | 3.0 |
| Cetyl alcohol | 2.5 |
| Preservative (Euxyl® K400) | 0.15 |
| Fragrance | 0.2 |
| Water | to 100 |
| pH, measured directly | 4.0 (adjusted with citric acid) |

Under stirring, Elfacos® CD 481 is wetted with water of minimally 70°C, the water optionally containing the cationic surfactant in an amount of about 1/3 of the total amount of water. When the powder is completely wetted, the rest of the water or surfactant solution is added and stirring is continued until the powder has completely dissolved. Then, the other ingredients are added with stirring.

The following two formulations were evaluated by a test panel, ingredients are given in weight percent:

| | | |
|---|---|---|
| Elfacos® CD 481 | 0.6 | -- |
| Hydroxyethyl cellulose (Natrosol® 250HHR) | -- | 0.6 |
| Cetrimonium chloride (Arquad® 16-25) | 3.0 | 3.0 |
| Cetearyl alcohol, Laureth-10 (Emulgade® A) | 0.5 | 0.5 |
| Isopropyl stearate | 3.0 | 3.0 |
| Cetyl alcohol (Lanette® 16) | 2.5 | 2.5 |
| Preservative (Euxyl® K400) | 0.15 | 0.15 |
| Fragrance (Parfümöl) | 0.2 | 0.2 |
| Demineralized water | to 100 | to 100 |

Half-head test: a panel of 7 people rated the above-mentioned ethyl hydroxyethyl cellulose-containing formulation significantly better than the reference formulation containing hydroxyethyl cellulose with regard to consistency of the formulation, wet-combing, wet feel, dry-combing, and dry feel, as shown below.

| Formulation containing | Elfacos® CD 481 | or Natrosol® 250HHR: |
|---|---|---|
| Appearance | 2.0 | 2.0 |
| Smell | 2.1 | 2.3 |
| Consistency | 2.0 | 2.6 |
| Ease of application | 2.0 | 2.0 |
| Rinsing out | 2.0 | 2.0 |
| Wet-combing | 1.6 | 2.1 |
| Wet feel | 1.6 | 2.3 |
| Blow-drying | 2.0 | 2.3 |
| Drying time | 2.0 | 2.0 |
| Dry-combing | 2.0 | 2.6 |
| Dry feel | 1.6 | 2.4 |
| Resilience | 2.0 | 2.0 |
| Shine | 2.0 | 2.0 |
| Volume | 2.1 | 2.0 |
| Ease of styling | 1.3 | 1.7 |
| Additional weight | 1.0 | 1.0 |

## Claims

1. Hair care formulation comprising a quaternary ammonium compound and a cellulose ether, characterized in that the cellulose ether is an ethyl hydroxyethyl cellulose ether.

2. Hair care formulation according to claim 1, characterized in that the ethyl hydroxyethyl cellulose ether has a degree of molar substitution of ethylene oxide of 1.5 to 3.5, an ethyl degree of substitution of 0.5-1.5, and an average degree of polymerization of 500 to 2,500.

3. Hair care formulation according to claim 1 or 2, characterized in that the ethyl hydroxyethyl cellulose ether is a hydrophobically modified ethyl hydroxyethyl cellulose ether.

4. Hair care formulation according to any one of the preceding claims, characterized in that the quaternary ammonium compound (quat) is a monoalkylquat of the formula RN(Me)₃⁺, a dialkylquat of the formula R₂N(Me)₂⁺ or a diesterquat of the formula (RC(O)OCH₂CH₂)₂N(Me)₂⁺ or (RC(O)OCH₂CH₂)₂N(Me)(CH₂CH₂OH)⁺, wherein R is a C₁₂-C₂₂ alkyl or a C₁₂-C₂₂ alkenyl group.
